Europäisches Patentamt

European Patent Office

Office européen des brevets

(11) Veröffentlichungsnummer: **0 340 774**

**A2**

(12) # EUROPÄISCHE PATENTANMELDUNG

(21) Anmeldenummer: 89108070.7

(22) Anmeldetag: 04.05.89

(51) Int. Cl.⁴: **A61B 5/10 , A61B 5/00**

(30) Priorität: 04.05.88 DE 3815096

(43) Veröffentlichungstag der Anmeldung:
08.11.89 Patentblatt 89/45

(84) Benannte Vertragsstaaten:
AT BE CH DE FR GB LI NL SE

(71) Anmelder: **Höll, Thomas**
**Hebelstrasse 11**
**D-7600 Offenburg(DE)**

(72) Erfinder: **Höll, Thomas**
**Hebelstrasse 11**
**D-7600 Offenburg(DE)**

(74) Vertreter: **Zahn, Roland, Dipl.-Ing.**
**Im Speitel 102**
**D-7500 Karlsruhe 41(DE)**

(54) Verfahren und Vorrichtung zur Messung elektrodermaler Reaktionen.

(57) Bei einem Verfahren und der entsprechenden Vorrichtung zur Messung elektrodermaler Reaktionen der Haut von Säugetieren, insbesondere von Menschen, für diagnostische Zwecke, wird einem auf der Hautoberfläche applizierten, nach außen hin dichten, geschlossenen Kanalsystem kontinuierlich ein Spülflüssigkeitsstrom zugeführt. In einer am Ausgang des Kanalsystems vorgesehenen, der Hautoberfläche gegenüber elektrisch isolierten Meßstrecke wird dabei die längs des Kanalsystems von der Hautoberfläche an den Spülflüssigkeitsstrom abgegebene Menge an Ionen elektrisch gemessen. Gemäß einer Weiterbildung wird gleichzeitig mit der Messung der Ionen im Kanalsystem auch die Menge des von der Hautoberfläche abgesonderten Schweißes gemessen.

Aufgrund der elektrischen Isolation der Meßstrecke gegenüber der Hautoberfläche erhält man so ein sehr genaues Meßsignal.

Fig.1

## Verfahren und Vorrichtung zur Messung elektrodermaler Reaktionen

Die vorliegende Erfindung bezieht sich auf ein Verfahren zur Messung elektrodermaler Reaktionen der Haut von Säugetieren, insbesondere von Menschen für diagnostische Zwecke. Die Erfindung bezieht sich weiterhin auf eine Vorrichtung zur Ausführung dieses Verfahrens.

Verfahren zur Messung elektrodermaler Reaktionen von Säugetieren, insbesondere von Menschen, d.h. zur quantitiven Bestimmung der über die Haut abgegebene Ionen und der über die Haut abgegebenen Wassermenge bei der Absonderung von Schweiß sind allgemein bekannt. Sie lassen sich grob und prinzipiell in zwei Bereiche untergliedern, und zwar

1. in Verfahren, bei denen die abgegebene Schweißmenge bestimmt wird, und

2. in Verfahren, bei denen die abgegebene Ionenmenge bestimmt wird.

Die die Schweißmenge bestimmenden Verfahren lassen sich ihrerseits in chemische und anderweitige Verfahren unterteilen.

Zu den chemischen Verfahren zählen hierbei die sogenannten Colorimetrischen Verfahren, bei denen durch den Hautschweiß eine Farbänderung einer aufgebrachten Substanz bewirkt wird (zum Beispiel Jod-Stärke-Reaktion). Ein weiteres chemisches Verfahren basiert auf dem semi-quantitativen Nachweis der Entstehung eines Chlorsilber-Bildes auf der Grundlage von Silbernitrat und Hautschweiß. Derartige Färbemethoden sind mehrfach bekannt und beschrieben (vergleiche Buch: Fiedler, H.P., "Der Schweiß", Aulendorf: Editio cantor, 1955, S. 42 ff.).

Der Nachteil dieser Färbeverfahren liegt darin, daß keine auch nur annähernd quantitative Bestimmung der emittierten Ionen (oder auch anderer quantitativer Parameter) möglich ist. Darüberhinaus sind Färbeverfahren nicht kontinuierlich anwendbar und sie sind vergleichsweise kompliziert und empfindlich.

Die anderweitigen Verfahren zur Bestimmung der Schweißmenge beruhen beispielsweise auf der Messung der Gewichtszunahme von Filterpapier oder anderen wasserabsorbierenden Substanzen, die auf die Haut aufgebracht werden. Mit weiteren Verfahren wurde versucht, die aus der Verdunstung des Schweißes zwischen Haut und Umgebung entstehenden Temperaturdifferenen zu messen und damit auf die Menge Schweiß zurückzuschließen; diese Verfahren konnten jedoch keine praktischen Bedeutung erlangen.

Die bekannten Methoden lassen sämtlich eine zeitliche Auflösung im Sekundenbereich vermissen, wie sie für wissenschaftliche Zwecke unerlässlich ist. Mit den bekannten Verfahren sind ferner keine kontinuierlichen Messungen möglich und außerdem kann hierbei auch nicht der Elektrolytgehalt des Schweißes, d.h. dessen Ionenkonzentration, bestimmt werden.

Es ist auch ein sogenanntes Taupunkt-Meßverfahren bekannt, bei dem ein in seiner Luftfeuchtigkeit und damit in seinem Taupunkt definierter Luftstrom über ein Meßgebiet geleitet und die Änderung der Meßfeuchte durch eine Veränderung des Taupunktes gemessen werden. Dieses kontinuierliche Meßverfahren ist jedoch sehr aufwendig und durch schwer zu beherrschende Parameter wie Turbulenzen in der Luft, Kondensationskeime und Temperaturschwankungen sehr fehleranfällig; auch ist mit diesem Taupunkt-Meßverfahren keine Erfassung von Leitfähigkeitsschwankungen des Schweißes im Sekundenbereich möglich.

Ein etwas präziseres Verfahren beruht auf der sogenannten Widerstandshygrometrie, bei der der Schweiß an eine in einem Sensor integrierte Substanz, wie zum Beispiel ein hygroskopisches Salz wie LiCl, LiSo4 oder CaCl 2 abgegeben wird. Diese hygroskopische Substanz ändert ihre elektrische Leitfähigkeit mit der Aufnahme des Schweißes, der vorzugsweise über einen Luftstrom von der Haut zum Sensor transportiert wird. Die Ausführung dieses an sich hinreichend genauen Verfahrens ist jedoch sehr aufwendig und wegen der erforderlichen Apparatur praktisch nur zur Messung bei gesunden Menschen beziehungsweise Tieren einsetzbar. Da der Schweißgehalt der hygroskopischen Substanz mit fortschreitender Meßdauer ansteigt, verändert sich der Sensor mit zunehmender Meßdauer bis hin zur Unbrauchbarkeit; die Grenze der Aufnahmefähigkeit ist dann erreicht. Die genannte Veränderung des Sensors durch die ständige Schweißaufnahme kann zwar entweder durch die Meßeinrichtung ausgeglichen oder im nachhinein durch rechnerische Korrektur kompensiert werden; dies ist jedoch mit großem Aufwand verbunden. Das Hygrometrie-Verfahren läßt zudem nur eine Aussage über die Menge des abgegebenen Wassers zu, nicht jedoch über die Menge der von der Haut emittierten Ionen.

Eines der genauesten Verfahren ist die Messung der Absorption von Infrarotstrahlen in einem strömenden Trägergas, welches von der Haut Schweiß aufnimmt. Mit diesem Verfahren von Johnson & Schuster (vergleiche Buch: Johnson, C. & Schuster, S.: "The measurement of transepidermal water loss"; Br.J.Derm. 1969, 81, 40-46) erreicht man überdies eine hohe zeitliche Auflösung und kann bestimmen, wieviel Schweiß pro Einheit Hautoberfläche abgegeben wurde. Dieses Verfahren bedingt jedoch den vergleichsweise größten apparativen Aufwand und ist daher in einer Forschungsarbeit mit Kranken oder nur gering belastbaren Menschen (oder Tieren) nicht anwendbar.

Eine der heute noch verbreiteten Methoden zur Messung der Menge der von der Haut emittierten Ionen, beziehungsweise des dieser Ionenemission entsprechenden Leitwertes, besteht im Aufbringen von zwei, vorzugsweise Ag/AgCl-Ektroden auf die Haut. Zwischen diesen beiden Elektroden fließt bei einer Meßspannung von cirka 0,5 Volt ein Meßstrom, dessen Veränderungen gemessen werden. Dieses an sich sehr kostengünstige und robuste Verfahren hat jedoch zahlreiche Nachteile. So lassen sich keinerlei quantitativen Aussagen machen, weil sich die Ergebnisse in Abhängigkeit von der Meßdauer, vom Elektrodengel, sowie von der Meßspannung verändern; darüberhinaus wird auch der Körperwiderstand selbst mitgemessen. Der Einfluß der verschiedenen Komponenten des Körperwiderstandes wird ausführlich bei Fowels diskutiert (vergleiche Buch: Fowels, D. C.: "Mechanisms of electrodermal activity"; In: Thopson, R.F. & Paterson, M.M. (ed.): Bioelectric recording techniques, New York / London: Academic Press, 1974).

Ein robustes und dabei sowohl quantitativ als auch zeitlich genau auflösendes Verfahren beruht auf einem kapazitiven Meßprinzip (Fa. Vaisala Stockholm, Evaporimeter Ep 1b der Va. Servomed / Stockholm, Schweden). Hierbei nimmt ein hochpolymerer Kunststoff den Schweiß auf, der gleichzeitig das Dieletrikum eines in einen elektrischen Schwingkreis integrierten Kondensators ist. Mit der Menge des aufgenommen Schweißes verändern sich auch die Dielektrizitätskonstante und damit die Parameter des Schwingkreises, wodurch zeitliche und quantitative Änderungen sehr genau erfaßt werden können.

Mit diesem, nach heutigem Stand der Technik besten Verfahren, können jedoch keine Aussagen über die Anzahl der emittierten Ionen beziehungsweise den Leitwert getroffen werden. Der absorbierte Schweiß verbleibt darüberhinaus zumindest für eine kurze Verweilzeit im Dielektrikum, so daß eine ständig wechselnde Fehlerquelle vorhanden ist. Der daraus resultierende Fehler muß durch die elektrische Schaltung an sich oder durch den die Meßdaten verarbeitenden Rechner eliminiert werden.

Der vorliegenden Erfindung liegt somit die Aufgabe zugrunde ein Verfahren der gattungsgemäßen Art anzugeben, mit dem die quantitative Registrierung elektrodermaler Reaktionen möglich ist, und zwar mit einem relativ geringen apparativen Aufwand und mit einer sehr hohen Meßgenauigkeit. Diese Meßgenauigkeit soll auch bei Langzeitmessungen aufrecht erhalten bleiben.

Der Erfindung liegt darüberhinaus auch die Aufgabe zugrunde, eine Vorrichtung zur Ausführung des genannten Verfahrens anzugeben.

Die erst genannte Aufgabe wird gemäß dem Grundgedanken der vorliegenden Erfindung durch die im kennzeichnenden Teil des Patentanspruchs 1 angegebene Verfahrensweise gelöst.

Mit anderen als den im Anspruch 1 gebrauchten Worten wird ein zur Hautoberfläche hin offenes Kanalsystem kontinuierlich mit einer Spülflüssigkeit durchspült, die dabei den abgesonderten Schweiß und die in diesem enthaltenen Ionen kontinuierlich zu einer Meßstrecke transportieren, die zur Haut elektrisch isoliert ist und zwei Elektroden aufweist, über die der Leitwert der Spülflüssigkeit und damit die Ionenkonzentration quantitativ bestimmt werden. Durch die elektrische Isolierung der Meßstrekke gegenüber der Hautoberfläche werden à priori Fehler eliminiert, die durch den Körper des Menschen beziehungsweise des Tiers bedingt sind und als zusätzlicher Widerstandswert, oder kapazitive und/oder induktive Komponente die Messung verfälschen wurden. Aufgrund der genannten elektrischen Isolierung ist es darüberhinaus möglich, Meßstrom, Spannung und Frequenz bei der Impedanzmessung über einen weiten Bereich zu variieren und damit zu optimieren.

Gerade diese Variations- beziehungsweise Optimierungsmöglichkeiten sind bei direkt an der Haut aufliegenden Elektrodenanschlüssen eingeschränkt, da hierbei unter Umständen unerwünschte elektrophysiologische Reaktionen ausgelöst würden. So ist es bekannt, daß sich der gesamte Hautwiderstand ab einer bestimmten Stromdichte nicht mehr linear verhält. Inwieweit eine angelegte Spannung bei direkt aufliegenden Elektroden die Schweißdrüsen anregt beziehungsweise hemmt, oder anderweitige Eigenschaften, wie zum Beipiel Membranladungen der Drüsen ändert, ist umstritten (vergleiche Buch: Fowels, D.C. - siehe oben -). Dieser die Messung störende Einfluß fällt jedoch beim erfindungsgemäßen Verfahren à priori gänzlich weg.

Die Tatsache, daß der Schweiß über ein Trägermedium, nämlich die Spülflüssigkeit der eigentlichen Messung zugeführt und schließlich körperfern durchgeführt wird, hat darüberhinaus den Vorteil, daß keine Elektrodencreme verwendet werden muß. Diese Elektrodencremes sind zwangsläufig mit Ionen angereichert und machen so eine quantitative Messung der Vorgänge unmöglich. Da die elektrischen Kontakte für die Impedanz-Meßeinrichtung also nicht direkt auf der Haut aufliegen, sind auch durch unterschiedliche Anpressdrücke bedingte Meßfehler ausgeschaltet, wie dies bei auf die Hautoberfläche aufgelegten Elektroden der Fall ist. Auch die durch Elektrodencremes initiierten chemischen Veränderungen der Haut fallen hier gänzlich weg. Damit reduzieren sich letztlich auch die sogenannten Einmeßzeiten, die bei über Elektrodencremes aufgelegten Metallelektroden bis zu etwa eine halbe Stunde betragen, auf den Sekundenbereich.

Der direkte Kontakt der Spülflüssigkeit mit dem Körper verhindert ferner den Verlust von Ionen und von Schweiß, wie es bei den Verfahren zu beobachten ist bei denen der Schweiß über einen Luftstrom zur Meßeinrichtung, d.h. zum Sensor transportiert wird.

Da das erfindungsgemäße Verfahren von einem Kanalsystem für die Spülflüssigkeit ausgeht, kann gemäß einer grundsätzlichen Weiterbildung des Verfahrens zur Messung der Ionenkonzentration durch Mengenmessung der zufließenden Spülflüssigkeitsmenge und durch Messen der einem Auffanggefäß zufließenden Spülflüssigkeitsmenge der Flüssigkeitszuwachs und damit die Menge des abgesonderten Schweißes bestimmt werden (Anspruch 2).

Wird so eine elektronische Wägung der Spülflüssigkeitsmenge vor dem Zulauf zum und nach dem Ablauf aus dem Kanalsystem in sehr kurzen Zeitabständen durchgeführt, so läßt sich die schnelle Änderung der abgesonderten Schweißmenge erfassen, indem man den Massenzuwachs im Auffangbehälter der Spülflüssigkeit mißt. Um diesen Massenzuwachs alleine in den Auffangbehälter gelangen zu lassen, ist eine Spülflüssigkeitspumpe vorgesehen, die einen unidirektionalen Fluß in Richtung des Auffangbehälters bewirkt. Die Messung der Massendifferenz erfolgt letztlich dadurch, daß sowohl das Ausgangsgefäß als auch der Auffangbehälter in einer Wägeelement Rechner-Meßkette erfaßt werden. Die im Rechner digitalisierten Werte bilden dann sowohl die Grundlage zur Messung des Massenzuwachses, als auch zur Kontrolle der Dichtigkeit des Kanalsystems. Bei einem Leck würde der Verlust von Spülflüssigkeit vom Rechner bemerkt.

Die Messung der elektrischen Eigenschaften der Spülflüssigkeit, sowie des Schweißzuwachses im Auffanggefäß kann bis in den Millisekundenbreich erfolgen und ist damit für wissenschaftliche Anwendungen hinreichend schnell.

Als Spülflüssigkeit wird vorzugsweise destilliertes Wasser verwendet (Anspruch 3).

Die Vorrichtung zur Ausführung des Verfahrens gemäß dem Grundgedanken, d.h. zur Messung der Ionenkonzentration in einem der Haut gegenüber elektrisch isolierten System, ist im kennzeichnenden Teil des Patentanspruchs 4 angegeben.

Diese Vorrichtung ist der Erweiterung des obigen Grundgedankens in Richtung auf die gleichzeitige Messung der Schweißmenge zufolge um die Meßeinrichtung zur Bestimmung der Schweißmenge ergänzt (Anspruch 5).

Weitere Ausgestaltungen und Modifikationen der Vorrichtung sind Gegenstand der weiteren Unteransprüche, wobei explizit nur darauf hingewiesen wird, daß das auf die Hautoberfläche aufzubringene Kunststoff-Formteil aus medizinischen beziehungsweise hygienischen Gründen als Einmalsensor, d.h. Wegwerfartikel, konzipiert ist.

Die Erfindung wird im folgenden anhand der Zeichnung naher erläutert. Diese zeigt in

Fig. 1 eine Prinzipdarstellung des Einmalsensors einschließlich der prinzipiellen Darstellung des Meßkreises zur Ionenmessung und des Versorgungskreises mit Meßkreis für die Spulflüssigkeit;

Fig. 2 eine Schnittdarstellung durch die Meßstrecke des Einmalsensors.

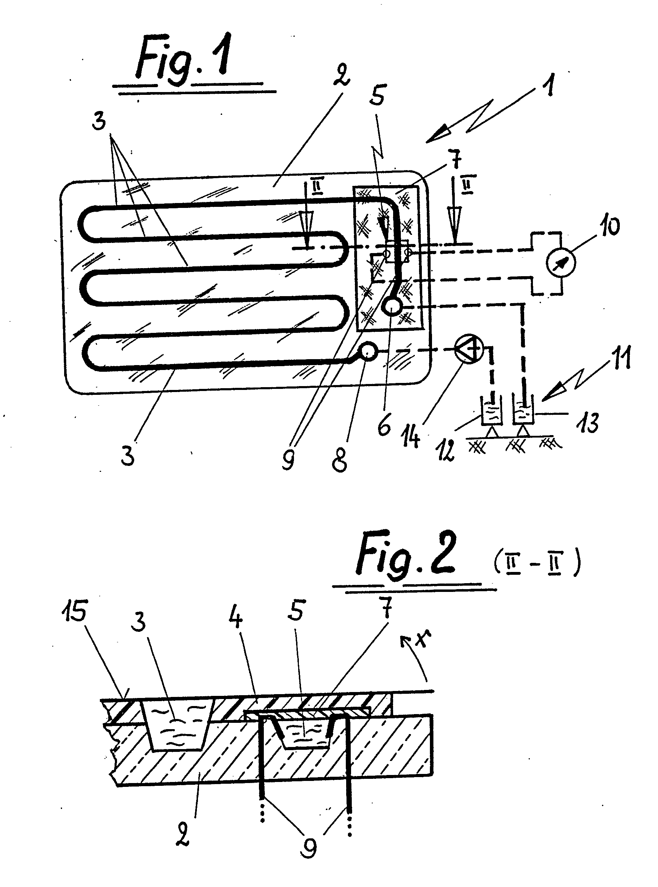

In Fig. 1 ist ein Einmalsensor in Form eines Kunststoff-Formteils 1 dargestellt, das im wesentlichen aus einem Kunststoff-Blättchen 2 besteht, dessen äußere Abmessungen etwa 10 mal 15 Millimeter betragen und dessen Dicke etwa 1 Millimeter beträgt. In dieses Kunststoff-Blättchen 2 ist einseitig eine ein Kanalsystem bildende mäanderförmige oder spiralförmige verlaufende Rille eingearbeitet, die beispielsweise eine Breite von 0,5 Millimeter und eine Tiefe von 0,35 Millimeter hat. Das Kunststoff-Blättchen ist unter Aussparung des Kanalsystems, d.h. der Rille 3 klebstoffbeschichtet (vergleiche Klebebeschichtung 4 in Fig. 2); die Rille 3 selbst ist im Bereich einer Meßstrecke 5 und einer Auslaßöffnung 6 für die Spülflüssigkeit durch eine nach der Applikation die elektrische Isolation zwischen der Haut und den Meßelektroden 9 bewirkende Kunststoff-Folie 7 abgedeckt und über dieser Kunststoff-Folie 7 ebenfalls klebstoffbeschichtet (vergleiche Fig. 2).

Die durch die Rille 3 geleitete Spülflüssigkeit, die - wie erwähnt - vorzugsweise destilliertes Wasser ist, wird über eine Einlaßöffnung 8 in das Kanalsystem eingeleitet. Bei der Applikation ist das Kunststoff-Formteil 1 mit seiner das offene Kanalsystem aufweisenden Fläche, d.h. mit der Klebebeschichtung 4 (Fig. 2) dicht auf die Hautoberfläche aufgelegt, so daß die Spülflüssigkeit im Bereich von der Einlaßöffnung 8 bis zur Kunststoff-Folie 7 zwischen den Rillen 3 des Kanalsystems und der Hautoberfläche fließt. Dabei nimmt die Spülflüssigkeit kontinuierlich sowohl die Ionen der (an der Hautoberfläche liegenden) ekrinen Schweißdrüsen auf, als auch die Volumenanteile des Schweißes selbst. Am Ende der Rille 3 und damit des Kanalsystems erfolgt dann, und zwar elektrisch durch die Kunststoff-Folie 7 und die Klebstoffbeschichtung 4 der Haut gegenüber isoliert mittels der zwei Elektroden 9 eine Messung des Wechselstrom-Widerstandes beziehungsweise der Impedanz. Zur Impedanzmessung selbst wird dabei ein handelsübliches Meßgerät 10 benutzt, das an die beiden Elektroden 9 angeklemmt ist und bei einer Meßspannung von 24 Volt oder weniger sinusförmige Meßsignale generiert, die ein Maß für die Ionenkonzentration in der Meßstrecke und damit in der Spülflüssigkeit sind.

Für die Applikation des Einmalsensors, d.h. des Kunststoff-Formteils 1 auf der Hautoberfläche wird eine Klebebeschichtung 4 aus modifiziertem Kautschuk verwendet. Dieser Klebstoff gewährleistet einerseits die Haftung des Einmalsensors auf der Haut und andererseits die Abdichtung des Kanalsystems, d.h. der Rille 3 relativ zueinander. Die Klebebeschichtung 4 hat dabei eine Dicke von beispielsweise cirka 0,15 Millimeter.

Ist das Kunststoff-Formteil 1 fest und dicht auf die Haut aufgebracht beziehungsweise aufgeklebt, so wird durch die Einlaßöffnung 8 Spülflüssigkeit in das Kanalsystem, d.h. die Rille 3 gepumpt; die Spülflüssigkeit tritt dann durch die Auslaßöffnung 6 wieder aus. Diese Auslaßöffnung 6 und die durch die Elektroden 9 bestimmte Meßstrecke zur Messung der Ionenkonzentration sind dabei wie erwähnt durch die Kunststoff-Folie 7 der Hautoberfläche gegenüber separiert.

Der im Vorstehenden erläuterte Einmalsensor ist nur zur Messung der Ionenkonzentration in der Spülflüssigkeit vor der Ableitung aus dem zwischen dem Kunststoff-Formteil 1 und der Haut einge-schlossenen Kanalsystem geeignet. In weiterer Ausgestaltung dieser Grundkonzeption ist vorgesehen, die durch das Kanalsystem fließende Spülflüssigkeit gleichzeitig auch zur Bestimmung der von der Hautoberfläche abgesonderten Schweißmenge zu benutzen. Dazu wird die Spülflüssigkeit zwischen der Einlaßöffnung 8 und der Auslaßöffnung 6, und zwar außerhalb des Kanalsystems, einer hochgenauen Wägeeinrichtung 11 zugeführt, mit der die Differenz zwischen der dem Kanalsystem zugeführten und der abgeführten Spülflüssigkeitsmenge bestimmt wird. In der Praxis wird diese Wägeaufgabe so gelöst, daß die bereitgestellte Spülflüssigkeit in einem Vorratsgefäß 12 gemessen wird und von hier über eine Pumpe 14, zum Beispiel eine Peristaltikpumpe, dem Kanalsystem zugeführt wird. Die über die Auslaßöffnung 6 abgeleitete Spülflüssigkeitsmenge wird dann einem Aufnahmegefäß 13 zugeführt. Die Mengendifferenz, die etwa bei 10 ... 100 Mykrogramm/h liegt wird dann bestimmt; sie ist ein Maß für die Menge angesonderten Schweißes. Gemäß der in Fig. 1 dargestellten Prinzipdarstellung wird die Spülflüssigkeit durch das Kanalsystem gepumpt. Es ist jedoch auch möglich, die Spülflüssigkeit durch das Kanalsystem zu saugen, was den Vorzug hat, daß Undichtigkeiten im Kanalsystem leichter erkennbar werden, da der Spülflüssigkeitsstrom dann abreißt. In diesem Falle ist in die Rückleitung eine optische und/oder akustische Alarmeinrichtung integrierbar, die - gegebenenfalls in Zusammenarbeit mit dem Auswerterechner - ein Alarmsignal generiert, wenn eine Undichtigkeit erfaßt wird.

Es liegt im Umfang der vorliegenden Erfindung, daß die Mengenmeßeinrichtung für den abgesonderten Schweiß auch durch ein in den Spülflüssigkeitsstrom integriertes Strömungsmengenmeßgerät realisiert sein kann.

Mit dem vorbeschriebenen Verfahren wird erstmals die Kombination, d.h. die gleichzeitige Erfassung beider Parameter "Ionenemssion" und "abgegebene Schweißmenge" ermöglicht. Die Fehlerquellen der bekannten Verfahren sind ausgeschaltet und die Verwendung des vorgeschlagenen Einmalsensors ist so einfach und unkompliziert, daß dieser im klinischen Routinebetrieb auch von Krankenschwestern gehandhabt werden kann. Die Person, welche sich der Messung unterziehen soll beziehungsweise das Tier, an dem die Messungen durchgeführt werden sollen, kann sich weitgehend uneingeschränkt bewegen; der Einmalsensor erfordert kaum besondere Rücksichtnahme. Dies ist vor allem in der psychiatrischen Forschung wichtig, da hier die zu untersuchenden Patienten nicht hinreichend ruhig gestellt sind, um die bekannten Verfahren einsetzen zu können.

Das Vergleichsweise kleine Kunststoff-Formteil läßt auch den Einsatz des Meßverfahrens bei Schwerverletzten und auch bei Neugeborenen zu, um dort den Flüssigkeits-und Elektrolytverlust über die Haut kontrollieren zu können. Die gleichzeitige Kontrolle der beiden oben genannten Parameter "Ionenemssion" und "abgesonderte Schweißmenge" ist mit keinem der bekannten Verfahren möglich; dem Patienten bleibt so eine zweite Meßvorrichtung am Körper erspart.

Die Dicke des Kunststoff-Formteils 1 einschließlich der Klebebeschichtung 4 liegt - je nach Fertigungstechnkik - bei cirka 2 Millimetern, so daß der Sensor die Versuchsperson oder das Versuchstier wenn überhaupt, nur wenig behindert.

Vor allem aber können Hand oder Finger eines Patienten bei psychophysiologischen Untersuchungen, bei welchen überlicherweise solche Sensoren angebracht werden, selbst bei mehreren applizierten Sensoren vergleichsweise frei bewegt werden, während bei den meisten bekannten Verfahren die Hand nicht bewegt werden darf, um die Messung nicht zu verfälschen.

Der im Vorstehden beschriebene Einmalsensor wird -bezüglich der Meßstrecke 5 zur Messung der Ionenkonzentration - anhand von Fig. 2 nochmals im vergrößerten Schnitt gezeigt.

Das Basis-Bauteil des Einmalsensors ist das Kunststoff-Blättchen 2 des Kunststoff-Formteils 1, in das das mäanderförmige (oder anderweitig gestaltete) Kanalsystem in Form der Rille 3 eingearbeitet ist. Die Rille 3 ist zur einen Fläche des Kunststoff-Formteils hin als offen. Diese Fläche ist mit einer Klebebeschichtung 4 überzogen, die einerseits die Rille 3 und damit das Kanalsystem ausspart, und die andererseits dazu dient, eine feste und dichte Auflage auf der Haut zu gewährleisten. Bei der Herstellung der Einmalsensoren der erfindungsgemäßen Art ist diese Klebebeschichtung 4 zusätzlich mit einer Schutzfolie 15 überzogen, die die Klebebeschichtung 4 vor der Austrocknung schützt und die unmittelbar vor der Applikation einfach abgezogen wird (vergleiche Pfeil X).

Wie anhand von Fig. 1 erläutert ist der Endbereich der Rille 3 bis hin zur Auslaßöffnung mit der Kunststoff-Folie 7 abgedeckt, die unmittelbar auf das Kunststoff-Formteil 2 aufgebracht und dann von der Klebebechichtung 4 überzogen ist. Über diese Kunststoff-Folie 7 wird die elektrische Isolation zwischen der Haut einerseits und der Meßstrecke 5, d.h. den Anschlüssen der Elektrode 9 an der Rille andererseits gewährleistet.

Zur Applikation selbst wird - wie erwähnt - die Schutzfolie 15 abgezogen, und der Sensor in Form des Kunststoff-Formteils 1 auf die Hautoberfläche aufgelegt beziehungsweise über die Klebebeschichtung 4 aufgeklebt. Anschließend werden die Elektroden 9 mit dem Impedanz-Meßgerät 10 und die Anschlüsse für die Spülflüssigkeits-Zu- und -Ablauf mit der Wägeeichrichtung 11 verbunden. Damit sind nun die Voraussetzungen für die bestimmungsgemäßen Messungen der Ionenemission einerseits und der Schweißmenge andererseits gegeben. Nach erfolgter Messung werden die genannten Verbindungen wieder aufgetrennt und der Sensor selbst wird entsorgt.

## Ansprüche

1. Verfahren zur Messung elektrodermaler Reaktionen der Haut von Säugetieren, insbesondere von Menschen, für diagnostische Zwecke,
dadurch gekennzeichnet,
daß einem auf der Hautoberfläche applizierten, nach außen hin dichten, geschlossenen Kanalsystem kontinuierlich ein Spülflüssigkeitsstrom zugeführt wird, und
daß in einer am Ausgang des Kanalsystems vorgesehenen, der Hautoberfläche gegenüber elektrisch isolierten Meßstrecke die längs des Kanalsystems von der Hautoberfläche an den Spülflüssigkeitsstrom abgegebene Menge an Ionen elektrisch gemessen wird.

2. Verfahren nach Anspruch 1,
dadurch gekennzeichnet,
daß gleichzeitig mit der Messung der Ionen im Kanalsystem die Menge des von der Hautoberfläche abgesonderten Schweißes gemessen wird.

3. Verfahren nach Anspruch 1 oder 2,
dadurch gekennzeichnet,
daß als Spülflussigkeit destilliertes Wasser verwendet wird.

4. Vorrichtung zur Ausführung des Verfahrens nach einem der Ansprüche 1 oder 3,
dadurch gekennzeichnet,
daß ein auf die Hautoberfläche aufbringbares Kunststoff-Formteil vorgesehen ist, das konstruktiv wie folgt ausgebildet ist:

a) Die auf die Hautoberfläche aufzulegende Fläche weist eine das Kanalsystem bildende offene Rille auf,

b) die Rille weist zur zweiten Fläche des Kunststoff-Formteils hin eine Einlaßöffnung zur Zuleitung der Spülflüssigkeit und eine Auslaßöffnung zur Ableitung der Spülflüssigkeit auf, und

c) im Verlauf der Rille ist vor der Auslaßöffnung zu beiden Seiten der Rille je ein Elektrodenanschluß vorgesehen, an die ein Meßgerät zur Bestimmung der Ionenkonzentration anschließbar ist.

5. Vorrichtung zur Ausführung des Verfahrens nach einem der Ansprüche 2 oder 3,
dadurch gekennzeichnet,
daß ein auf die Hautoberfläche aufbringbares Kunststoff-Formteil vorgesehen ist, das konstruktiv wie folgt ausgebildet ist:

a) Die auf die Hautoberfläche aufzulegende Fläche weist eine das Kanalsystem bildende offene Rille auf,

b) die Rille weist zur zweiten Fläche des Kunststoff-Formteils hin eine Einlaßöffnung zur Zuleitung der Spülflüssigkeit und eine Auslaßöffnung zur Ableitung der Spülflüssigkeit auf,

c) im Verlauf der Rille ist vor der Auslaßöffnung zu beiden Seiten der Rille je ein Elektrodenanschluß vorgesehen, an die ein Meßgerät zur Bestimmung der Ionenkonzentration anschließbar ist, und

d) das von der Rille gebildete Kanalsystem ist über die Einlaß- und Auslaßöffnung mit einer Meßeinrichtung zur Messung der von der Hautoberfläche abgesonderten Menge Schweiß gekoppelt.

6. Vorrichtung nach einem der Ansprüche 4 oder 5,
dadurch gekennzeichnet,
daß die elektrische Isolierung der Meßstrecke für die Ionenkonzentration durch eine auf das Kunststoff-Formteil aufgeklebte, die Meßstrecke und die Auslaßöffnung überdeckende wasserdichte, hautneutrale Kunststoff-Folie gebildet ist.

7. Vorrichtung nach einem der Ansprüche 4 bis 6,
dadurch gekennzeichnet,

daß das Kunststoff-Formteil auf der das Kanalsystem aufweisenden Fläche von einer die Rille aussparenden Klebeschicht überzogen ist.

8. Vorrichtung nach einem der Ansprüche 4 bis 7,
dadurch gekennzeichnet,
daß die Rille mäanderförmig verläuft.

9. Vorrichtung nach einem der Ansprüche 4 bis 7,
dadurch gekennzeichnet,
daß die Rille spiralförmig verläuft.

10. Vorrichtung nach einem der Ansprüche 4 bis 7,
dadurch gekennzeichnet,
daß sie durch einen an das Meßgerät zur Bestimmung der Ionenkonzentration und gegebenenfalls die Meßeinrichtung zur Messung der Schweißmenge ankoppelbaren Einmalsensor gebildet ist.

11. Vorrichtung nach einem der Ansprüche 4 bis 10,
dadurch gekennzeichnet,
daß das Meßgerät zur Bestimmung der Ionenkonzentration ein Impedanz-Meßgerät ist.

12. Vorrichtung nach einem der Ansprüche 4 bis 11,
dadurch gekennzeichnet
daß die Meßeinrichtung zur Bestimmung der Schweißmenge eine Wägeeinrichtung zur Mengendifferenzmessung der Spülflüssigkeit zwischen Zu- und Ablauf ist.

13. Vorrichtung nach einem der Ansprüche 4 bis 11,
dadurch gekennzeichnet,
daß die Meßeinrichtung zur Bestimmung der Schweißmenge ein im Spülflüssigkeitsstrom integriertes Strömungsmengenmeßgerät ist.

14. Vorrichtung nach einem der Ansprüche 4 bis 13,
dadurch gekennzeichnet,
daß zur Zuführung des Spülmittelstromes eine Perisaltikpumpe vorgesehen ist.

15. Vorrichtung nach Anspruch 14,
dadurch gekennzeichnet,
daß die Peristaltikpumpe so geschaltet ist, daß die Spülflüssigkeit von der Einlaß- zur Auslaßöffnung gepumpt wird.

16. Vorrichtung nach Anspruch 14,
dadurch gekennzeichnet, daß die Perisaltikpumpe so geschaltet ist, daß die Spülflüssigkeit an der Auslaßöffnung angesaugt wird.

7. Vorrichtung nach einem der Ansprüche 4 bis 16,
dadurch gekennzeichnet,
daß auf das Kunststoff-Formteil auf der auf die Hautoberfläche aufzuliegnden Fläche eine abziehbare Schutzfolie aufgebracht ist.

Fig.1

Fig.2 (II - II)